# EUROPÄISCHE PATENTSCHRIFT

(11) **EP 1 882 492 B1**
(45) Veröffentlichungstag und Bekanntmachung des Hinweises auf die Patenterteilung: **22.10.2008**
(21) Anmeldenummer: 06015462.2
(22) Anmeldetag: 25.07.2006
(51) Int. Cl.: A61M 37/00, A61B 5/053

(54) **Vorrichtung zum kontrollierten Einstechen in ein Objekt und Verfahren zum Betreiben der Vorrichtung**
Device for the controlled penetration into an object and method of operating the same
Dispositif pour une pénétration controlée dans un objet et procédé pour faire fonctionner ce dispositif

(43) Veröffentlichungstag der Anmeldung: 30.01.2008
(73) Patentinhaber: MT Derm GmbH, 14167 Berlin (DE)
(72) Erfinder: Kluge, Jörn, 14513 Teltow (DE); Lehr, Heinz Prof., 14197 Berlin (DE)
(74) Vertreter: Bittner, Thomas L.

(56) Entgegenhaltungen:
- EP-A1- 1 495 782
- WO-A-00/09184
- GB-A- 2 335 990
- US-B1- 6 520 927

## Beschreibung

Die Erfindung betrifft eine Vorrichtung zum kontrollierten Einstechen in ein Objekt und Verfahren zum Betreiben der Vorrichtung.

### Hintergrund der Erfindung

Vorrichtungen zum kontrollierten Einstechen in ein Objekt werden zum Beispiel dazu genutzt, einen Wirkstoff in das Objekt zu injizieren. Der Begriff Wirkstoff ist hier in sehr allgemeiner Form zu verstehen. Es kann sich bevorzugt um einen medizinischen oder einen kosmetischen Wirkstoff handeln. Hierzu gehören Impfstoffe jeglicher Art ebenso wie Farbstoffe, zum Beispiel Tätowierfarbstoffe oder Farbstoffe für permanentes Make-up.

Durch die Injektion spezieller DNA-Impfstoffe in die Oberfläche der menschlichen Haut lässt sich eine T- und B-Zellen-Immunität herbeiführen. Die Antigene eignen sich als Impfstoffe gegen verschiedene Tumore, Grippeviren sowie HIV (vgl. Bins et al., Nature Medicine 1264, Juni 2005). Bei dem Impfprozess wird das Antigen durch eine Reihe von intrakutan Injektionen in die Epidermis eingebracht. Ziel ist es hierbei, eine Vielzahl von Langerhans'schen Zellen zu benetzen, die eine Vermittlerrolle bei Immunisierungsprozessen einnehmen. Es werden typisch einige tausend Einstiche mit einer oder mehreren Injektionsnadeln auf einer Fläche von wenigen Quadratzentimetern beispielsweise am Oberarm durchgeführt, um sicher zu stellen, dass ausreichend Wirkstoff für den Impfprozess appliziert wurde. Von besonderer Bedeutung ist hierbei, dass der Wirkstoff in einen bestimmten Bereich der Hautschichten eingebracht wird, um die gewünschte Wirkung zu erzielen. Dieses hängt insbesondere von einer korrekten Einstechtiefe des Stechmittels ab.

Bei der Nutzung der Vorrichtung zum kontrollierten Einstechen in eine Haut als Tätowier- oder Permanent-Make-up-Gerät erfolgt ein Einstechen bis in den Bereich der Dermis. Bei anderen Anwendungen kann ein noch tiefergehendes Einstechen vorgesehen sein.

Verfahren zum Messen einer Einstechtiefe in ein Objekt, insbesondere eine Haut, sind als solche in verschiedenen Ausführungsformen bekannt. In dem Dokument WO 2004 / 080306 wird zur Durchführung sensorischer Messungen eine Messeinrichtung zum Messen einer Einstechtiefe vorgeschlagen, bei der ein Kontaktring auf der Haut als erste Elektrode dient, die zusammen mit einer in die Haut eindringenden Mikronadel zur Impedanzmessung genutzt werden soll. Dabei dient die Impedanzänderung als Funktion der Eindringtiefe in die Haut als Messwert. Je nach Hauttyp und Art der Elektrode ist jedoch sowohl der Absolutwert der Impedanz als auch das Verhalten der Impedanz als Funktion der Eindringtiefe sehr unterschiedlich, so dass eine solche Messmethode für den Impfprozess scheitern muss. Es wurde vorgeschlagen, eine zusätzliche Vergleichselektrode auf der Haut zur Normierung einzusetzen, um wenigstens die Unsicherheit des Hautkontakts auszuschalten.

In US 6,520,927 wird ein Verfahren zur Behandlung von Herz-Rhythmus-Störungen offenbart. Das Verfahren wird mittels einer Vorrichtung durchgeführt, die einen Katheter und eine in dem Katheter angeordnete Nadel umfaßt. Mittels der Nadel werden Hohlräume in den Herzwänden geschaffen, in denen dann Material mit einer gewünschten elektrischen Eigenschaft injiziert wird.

GB 2,335,990 A offenbart eine Vorrichtung für eine Blutentnahme. Die Vorrichtung umfaßt eine Nadel, in welcher ein leitfähiger Draht bis zu einem distalen Ende der Spitze verläuft. Mittels einer leitfähigen Beschichtung der Außenseite der Spitze sind somit zwei Elektroden gebildet, welche für eine Impedanzmessung zur Bestimmung einer Einstechtiefe der Nadel verwendet werden können.

WO 00/09184 beschreibt eine Vorrichtung für die Injektion einer Substanz. Die Vorrichtung weist eine oder mehrere Nadeln auf, welche mit einer hohen Geschwindigkeit in eine Haut eingestochen werden.

Aus EP 1,495,782 A1 ist eine Vorrichtung zum Aufbringen einer Tätowierung oder von permanentem Make-up mit einem Einwegmodul und einem Handstück bekannt, wobei das Einwegmodul von dem Handstück abnehmbar ist.

### Zusammenfassung der Erfindung

Aufgabe der Erfindung ist es, eine Vorrichtung zum kontrollierten Einstechen in ein Objekt, insbesondere zum kontrollierten Aufstechen einer Haut, und ein Verfahren zum Betreiben der Vorrichtung zu schaffen, bei denen die Nutzungseigenschaften für den Anwender verbessert sind.

Diese Aufgabe wird erfindungsgemäß durch eine Vorrichtung nach dem unabhängigen Anspruch 1 sowie ein Verfahren nach dem unabhängigen Anspruch 21 gelöst.

Mit der Erfindung ist dem Anwender die Möglichkeit gegeben, das Einstechen in ein Objekt auf definierte Art und Weise unter Einhaltung von Anwendungsvorgaben bei der Nutzung der Vorrichtung auszuführen. Die Vorrichtung ermöglicht es, in der Messbetriebsart eine Einstechtiefe der Stechmittel zu ermitteln. In Abhängigkeit von der gemessenen Einstechtiefe kann die Vorrichtung dann so gesteuert werden, dass eine Ausfahramplitude der Stechmittel eingestellt wird, die ihrerseits eine vorgegebene Einstechtiefe sicherstellt. Für die Messung der Einstechtiefe in der Messbetriebsart können beliebige Messverfahren zum Ermitteln der Einstechtiefe genutzt werden, wie sie als solche beispielsweise zur Messung der Einstechtiefe in eine Haut bekannt sind. Die Vorrichtung kombiniert somit die Vorteile einer exakten Messung der Einstechtiefe der Stechmittel mit der gezielten Steuerung der Ausfahramplitude der Vor- / Rückwärtsbewegung der Stechmittel in der Anwendungsbetriebsart.

Bei einer vorteilhaften Ausgestaltung der Erfindung ist vorgesehen, dass ein Reservoir mit einer eine Wirkstoff enthaltenden Flüssigkeit gebildet ist, welches mit den Stechmitteln durch eine Fluidverbindung verbunden ist, über die die Flüssigkeit zumindest in einer Anwendungsbetriebsart zur kontrollierten Wirkstoffabgabe zu den Stechmitteln gelangt. Hierdurch ist die Möglichkeit geschaffen, das kontrollierte Abgeben einer Flüssigkeit mit einem Wirkstoff in definierter Art und Weise in Abhängigkeit von einer Einstechtiefe der Stechmittel auszuführen.

Eine zweckmäßige Fortbildung der Erfindung sieht vor, dass die Messeinrichtung konfiguriert ist, um in der Messbetriebsart Messwerte für eine Einstechtiefe der Stechmittel in das Objekt gemäß zumindest einer der folgenden Messarten zu ermitteln: Einzeleinstichmessung und Messung von mehreren Einstichen bei repetierenden Vor- / Rückwärtsbewegungen der Stechmittel.

Eine bevorzugte Weiterbildung der Erfindung sieht vor, dass die Fluidverbindung eine gepulst betreibbare Fluidverbindung ist, die konfiguriert ist, um in Abhängigkeit von einer Bewegungsfrequenz der repetierenden Vor- / Rückwärtsbewegung der Stechmittel die Flüssigkeit in Teilmengen aus dem Reservoir zu den Stechmitteln zu liefern.

Bei einer zweckmäßigen Ausgestaltung der Erfindung kann vorgesehen sein, dass die Stechmittel durch einen Durchbruch in einer Wandung des Reservoirs geführt sind, in dem die Stechmittel bei der repetierenden Vor- / Rückwärtsbewegung geführt sind.

Eine vorteilhafte Ausführungsform der Erfindung sieht vor, dass eine Pumpeinrichtung, die konfiguriert ist, um die Flüssigkeit aus dem Reservoir zu den Stechmitteln zu pumpen.

Bevorzugt sieht eine Fortbildung der Erfindung vor, dass die Steuereinrichtung an die Pumpeinrichtung gekoppelt und konfiguriert ist, um einen die Pumpeinrichtung anregenden Pumpimpuls an die Pumpeinrichtung abzugeben, welcher einer vorgegebenen Ausfahramplitude einer ausgefahrenen Stellung der Stechmittel bei der repetierenden Vor- / Rückwärtsbewegung der Stechmittel zugeordnet ist.

Bei einer vorteilhaften Ausgestaltung der Erfindung kann vorgesehen sein, dass die Steuereinrichtung konfiguriert ist, um den Pumpimpuls an die Pumpeinrichtung abzugeben, wenn die vorgegebene Amplitude einer maximal ausgefahrenen Stellung der Stechmittel entspricht.

Eine Weiterbildung der Erfindung kann vorsehen, dass die Pumpeinrichtung eine Mikropumpe umfasst.

Eine bevorzugte Weiterbildung der Erfindung sieht vor, dass die Fluidverbindung einen Kanal in den Stechmittel umfassen, welcher zu einer an den Stechmitteln gebildeten Abgabeöffnung führt.

Bei einer zweckmäßigen Ausgestaltung der Erfindung kann vorgesehen sein, dass die Messeinrichtung eine Impedanzmesseinrichtung zum Messen einer Impedanz des Objektes ist.

Eine vorteilhafte Ausführungsform der Erfindung sieht vor, dass die Stechmittel eine oder mehrere an die Messeinrichtung gekoppelte Messelektroden umfassen.

Bevorzugt sieht eine Fortbildung der Erfindung vor, dass mehrere Messelektroden jeweils durch eine von den Stechmitteln umfasste Nadel gebildet sind.

Bei einer vorteilhaften Ausgestaltung der Erfindung kann vorgesehen sein, dass eine Messelektrode mittels der Stechmittel und eine Messgegenelektrode von einem Gehäuseteil gebildet sind.

Eine Weiterbildung der Erfindung kann vorsehen, dass die Stecheinrichtung in einem von der Antriebseinrichtung lösbaren Einwegmodul gebildet ist.

Eine bevorzugte Weiterbildung der Erfindung sieht vor, dass durch einen elektrischen Impulsgenerator, welcher mit den Stechmitteln elektrisch verbunden ist und konfiguriert ist, um einen elektrischen Impuls auf die Stechmittel zu einem vorgegebenen Zeitpunkt zu geben. Eine Amplitude des elektrischen Impulses ist regelbar. Bei der Verwendung der Vorrichtung zum Hautaufstechen kann diese so groß gewählt werden, dass eine Hautstimulierung bis hin zu einer Koagulation der Haut erreicht wird.

Bei einer zweckmäßigen Ausgestaltung der Erfindung kann vorgesehen sein, dass die Antriebseinrichtung einen Wandlungsmechanismus umfasst, welcher konfiguriert ist, um eine Drehbewegung eines Elektromotors in eine auf die Stechmittel einzuleitende repetierende Schubbewegung zu wandeln.

Eine vorteilhafte Ausführungsform der Erfindung sieht vor, dass der Wandlungsmechanismus einen Taumelscheibenanordnung umfasst, bei der eine Taumelscheibe an eine Antriebswelle des Elektromotors und an die Taumelscheibe ein Kopplungsmechanismus gekoppelt sind, wobei der Kopplungsmechanismus konfiguriert ist, um die repetierende Schubbewegung auf die Stechmittel einzuleiten.

Bevorzugt sieht eine Fortbildung der Erfindung vor, dass der Kopplungsmechanismus einen an die Taumelscheibe gekoppelte Stößel aufweist, welcher in einer Linearführung geführt ist.

Bei einer vorteilhaften Ausgestaltung der Erfindung kann vorgesehen sein, dass das Reservoir in einer Stechmitteldüse gebildet ist. In Kombination hiermit oder alternativ hierzu sieht eine Ausführungsform der Erfindung vor, dass das Reservoir mittels eines an dem Handgerät lösbar befestigten und hierdurch austauschbaren, sei es beispielsweise zum Nachfüllen oder zur Änderung des Reservoirvolumens, Reservoirbehälters gebildet ist, welcher an einen Teilabschnitt der Fluidverbindung angeschlossen ist, indem der Reservoirtank zum Beispiel auf ein Anschlussstück aufgesteckt oder hieran angeschraubt ist. Der Reservoirtank Ist vorzugsweise als Einwegtank ausgeführt. Obwohl das Vorsehen des Reservoirs bevorzugt ist, kann in einer vereinfachten Ausgestaltung ein solches Reservoir weggelassen werden. Die abzugebende Flüssigkeit gelangt an die Stechmittel, indem diese in einen Flüssigkeitsvorrat eingetaucht werden. Dieses Eintauchen wird dann während der Nutzung der Vorrichtung mehrfach wiederholt. Beim Eintauchen verteilt sich die abzugebende Flüssigkeit zumindest entlang der äußeren Oberfläche der Stechmittel.

Weiterhin können bei der Erfindung bevorzugte Ausgestaltungen des Verfahrens gebildet sein, die nachfolgend näher erläutert werden.

Bei einer Ausführungsform der Erfindung ist vorgesehen, dass die Flüssigkeit aus einem Reservoir über eine Fluidverbindung zu den Stechmitteln geführt wird.

Eine Fortbildung der Erfindung sieht vor, dass die Einstechtiefe in der Messbetriebsart gemäß zumindest einer der folgenden Messarten gemessen wird: Einzeleinstichmessung und Messung von mehreren Einstichen bei repetierenden Vor- / Rückwärtsbewegungen der Stechmittel.

Eine Weiterbildung der Erfindung kann vorsehen, dass die Messbetriebsart abgebrochen wird, nachdem aus den gemessenen Messwerten die vorgegebene Einstechtiefe ermittelt wurde.

Eine bevorzugte Weiterbildung der Erfindung sieht vor, dass die Flüssigkeit in Abhängigkeit von einer Bewegungsfrequenz der repetierenden Vor- / Rückwärtsbewegung der Stechmittel über eine gepulste Fluidverbindung in Teilmengen aus dem Reservoir zu den Stechmitteln geliefert wird.

Bei einer zweckmäßigen Ausgestaltung der Erfindung kann vorgesehen sein, dass die Flüssigkeit unter Ausnutzung von Adhäsionskräften entlang eines Oberflächenabschnittes der Stechmittel gerührt wird, indem die Stechmittel durch einen Durchbruch in einer Wandung des Reservoirs geführt sind, in welchem die Stechmittel bei der repetierenden Vor- / Rückwärtsbewegung geführt werden.

Eine vorteilhafte Ausführungsform der Erfindung sieht vor, dass die Flüssigkeit mittels einer Pumpeinrichtung aus dem Reservoir zu den Stechmitteln gepumpt wird.

Bevorzugt sieht eine Fortbildung der Erfindung vor, dass die Steuereinrichtung an die Pumpeinrichtung gekoppelt und einen die Pumpeinrichtung anregenden Pumpimpuls an die Pumpeinrichtung abgibt, welcher einer vorgegebenen Ausfahramplitude einer ausgefahrenen Stellung der Stechmittel bei der repetierenden Vor- / Rückwärtsbewegung der Stechmittel zugeordnet ist.

Bei einer vorteilhaften Ausgestaltung der Erfindung kann vorgesehen sein, dass die Steuereinrichtung den Pumpimpuls an die Pumpeinrichtung abgibt, wenn die vorgegebene Ausfahramplitude einer maximal ausgefahrenen Stellung der Stechmittel entspricht.

Eine Weiterbildung der Erfindung kann vorsehen, dass in der Messbetriebsart die Messwerte für die Einstechtiefe als Impedanzmesswerte des Objektes erfasst werden.

Eine bevorzugte Weiterbildung der Erfindung sieht vor, dass mittels eines elektrischen Impulsgenerators, welcher mit den Stechmitteln elektrisch verbunden ist, ein elektrischer Impuls auf die Stechmittel zu einem vorgegebenen Zeitpunkt gegeben wird, wodurch an den Stechmitteln eine hohe elektrische Feldstärke erzeugt wird, wobei ein Impulsabgabezeitpunkt einer weiteren vorgegebenen Ausfahramplitude in einer weiteren ausgefahrenen Stellung der Stechmittel bei der repetierenden Vor- / Rückwärtsbewegung der Stechmittel zugeordnet wird, wobei die weitere vorgegebene Ausfahramplitude wahlweise mit der vorgegebnen Ausfahramplitude übereinstimmt. Es kann vorgesehen sein, dass die vorgegebne Ausfahramplitude und / oder die weitere vorgegebne Ausfahramplitude der der vorgegebenen Einstechtiefe zugeordneten Ausfahramplitude entsprechen. Auch wenn der elektrische Impulsgenerator bevorzugt in die Vorrichtung zur kontrollierten Flüssigkeitsabgabe integriert wird, so kann alternativ eine Vorrichtung vorgesehen sein, in welcher der Impulsgenerator mit den Bauteilen zur Einstechtiefen-Messung und zur Einstellung einer hiervon abhängigen Ausfahramplitude kombiniert ist, ohne dass eine Flüssigkeitsabgabe und somit die hierfür genutzten Mittel ausgebildet sind. In analoger Weise werden in einer Impulsabgabe-Betriebsart elektrische Impulse auf die sich repetierend bewegenden Stechmittel gegeben.

Bei einer zweckmäßigen Ausgestaltung der Erfindung kann vorgesehen sein, dass in der Antriebseinrichtung mit einem Wandlungsmechanismus eine Drehbewegung eines Elektromotors in eine auf die Stechmittel einzuleitende repetierende Schubbewegung umgewandelt wird.

### Beschreibung bevorzugter Ausführungsformen der Erfindung

Die Erfindung wird im folgenden anhand von bevorzugten Ausführungsformen unter Bezugnahme auf Figuren einer Zeichnung näher erläutert. Hierbei zeigen:
- Fig. 1: eine schematische Darstellung einer Vorrichtung zum kontrollierten Abgeben einer einen Wirkstoff enthaltenden Flüssigkeit;
- Fig. 2: eine schematische Darstellung eines Stechwerkzeugs mit hieran gekoppelter Messeinrichtung zum Messen der Einstechtiefe eines Stechmittels;
- Fig. 3: einen Taumelscheibenantrieb im Querschnitt;
- Fig. 4: eine schematische Darstellung eines Handgeräts mit einem Stechwerkzeug und einer Antriebseinrichtung;
- Fig. 5: eine schematische Darstellung eines Stechwerkzeugs mit hieran gekoppelter Messeinrichtung zum Messen einer Einstechtiefe eines Stechmittels; und
- Fig. 6: eine schematische Darstellung eines weiteren Stechwerkzeugs mit hieran gekoppelter Messeinrichtung zum Messen der Einstechtiefe eines Stechmittels.

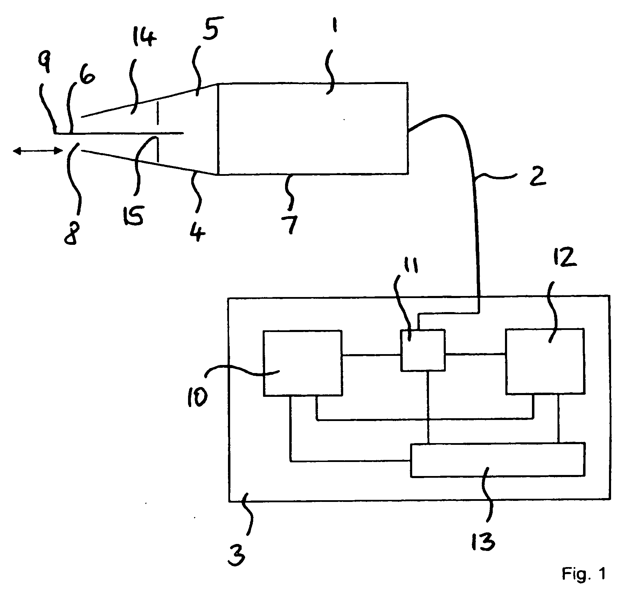

Fig. 1 zeigt eine schematische Darstellung einer Vorrichtung zum kontrollierten Einstechen in ein Objekt, vorzugsweise unter Einbeziehung einer kontrollierte Abgabe einer einen Wirkstoff enthaltenden Flüssigkeit. Eine Handgerät 1, welches beim Abgeben der Flüssigkeit von einem Benutzer mit der Hand geführt wird, ist über ein Verbindungskabel 2, welches in einer alternativen Ausgestaltung auch als kabellose Verbindung ausgeführt sein kann, mit einem Steuergerät 3 verbunden. Über das Verbindungskabel 2 wird das Handgerät 1 einerseits mit einer elektrischen Betriebsspannung versorgt, die üblicherweise regelbar ist, und andererseits werden zwischen dem Handgerät 1 und dem Steuergerät 3 elektronische Daten ausgetauscht. Letztere umfassen insbesondere Messdaten und Steuerdaten.

Das Handgerät 1 umfasst als Grundkomponenten ein Stechwerkzeug 4 mit einer Aufnahme 5, in welcher ein Stechmittel 6 verschiebbar gelagert ist. Bei dem Stechmittel 6 handelt es sich um eine Einzelnadel oder ein Nadelsystem mit zwei oder mehreren Nadeln. Das Stechwerkzeug 4 ist an eine Antriebseinrichtung 7 gekoppelt, die dann dazu dient, das Stechmittel 6 repetierend vor und zurück zu bewegen. Hierbei bewegt sich das Stechmittel 6 durch eine Öffnung 8, wobei eine Spitze 9 des Stechmittels 6 in einer vollständig eingefahrenen Stellung sich vor oder hinter der Öffnung 8 befinden kann. Eine Ausfahramplitude des Stechmittels 6, d. h. die Länge eines Abschnitts des Stechmittels 6 außerhalb der Öffnung 8 in einer vollständig ausgefahrenen Stellung des Stechmittels 6, kann dadurch eingestellt werden, dass entweder ein Hub des Stechmittels 6 oder eine Position der Öffnung 8 in Längsrichtung des Stechmittels 6 verändert wird. Letzteres wird mit einer Verlagerung der Aufnahme 5 erreicht. Auch eine Kombination dieser beiden Einstellmöglichkeiten zur Definition der Ausfahramplitude kann vorgesehen sein.

In dem Steuergerät 3 ist eine Messeinrichtung 10 gebildet, die über eine Schnittstelle 11 an das Verbindungskabel 2 und hierdurch an das Handgerät 1 gekoppelt ist. In der schematischen Darstellung in Fig. 1 ist die Messeinrichtung 10 als Block in das Steuergerät 3 integriert. Es versteht sich, dass Elemente einer die Messeinrichtung 10 in Fig. 1 umfassenden Messanordnung auch in das Handgerät 1 integriert sein können, beispielsweise Elektroden für einen Kontakt mit dem zu messenden Objekt. Mit der Messanordnung wird die Einstechtiefe des Stechmittels 6 in das Objekt erfasst. Dieses wird unten im Detail erläutert.

Das Steuergerät 3 umfasst weiterhin eine Steuereinrichtung 12, welche an die Schnittstelle 11 und die Messeinrichtung 10 angeschlossen ist. Die Steuereinrichtung 12 dient insbesondere dazu, in einer zum Beispiel als Wirkstoffabgabe-Betriebsart bezeichneten Anwendung, eine vorbestimmte Ausfahramplitude des Stechmittels 6 einzustellen, welche eine vorgegebene Einstechtiefe realisiert. Die so eingestellte Ausfahramplitude kann größer oder kleiner als die in der Meßbetriebsart gemessenen Einstechtiefe sein oder dieser entsprechen.

Die Größe der in der Wirkstoffabgabe-Betriebsart eingestellten Ausfahramplitude wird von der Steuereinrichtung 12 in Abhängigkeit von den Messwerten für die Einstechtiefe in einer vorangehenden Messbetriebsart festgelegt. Hierbei kann ein Benutzer des Steuergeräts 3 über eine Bedieneinrichtung 13, beispielsweise eine Tastatur oder eine berührungssensitive Anzeige, gewünschte Vorgaben für die Flüssigkeitsabgabe definieren, beispielsweise die Auswahl einer bestimmten Hautschicht, in welche die Flüssigkeit mit dem Wirkstoff eingebracht werden soll. Ausgehend von einer solchen Vorgabe ist die Steuereinrichtung 12 konfiguriert, um unter Berücksichtigung der Messergebnisse in der Messbetriebsart eine Ausfahramplitude einzustellen, die eine der Benutzereinstellung zuzuordnende Einstechtiefe entspricht. Beispielsweise kann dieses derart erfolgen, dass einer vom Benutzer ausgewählten Hautschicht ein bestimmtes Messergebnis für die Einstechtiefe zugeordnet ist. Wenn dieses Messergebnis dann in der Messbetriebsart bei der Ermittlung der momentanen Einstechtiefe auftritt, erkennt die Steuereinrichtung 12 die hierzu gehörige Ausfahramplitude und stellt diese für die Wirkstoffabgabe-Betriebsart ein.

Die Bewegung des Stechmittels wird durch eine periodische Hubbewegung veranlasst, die mittels der Antriebseinrichtung 7 erzeugt wird, welche einen elektrischen Motor zum Erzeugen einer Antriebsbewegung umfasst.

In einer Ausführung ist die Aufnahme 5 aus Metall und gewölbt ausgeführt. Im Betrieb wird das Handgerät 1 auf das zu stechende Objekt, beispielsweise eine Hautoberfläche, aufgesetzt sowie im Kontakt mit der Oberfläche des Objektes senkrecht dazu bewegt, so dass bei der oszillatorischen Hubbewegung des Stechmittels 6 aus dem Handgerät 1 heraus die Flüssigkeit durch viele Stechvorgänge in beliebig großen Flächen des Objektes appliziert wird. Im einfachsten Fall wird die Flüssigkeit durch die Bewegung des Stechmittels 6 ausgebracht, indem sich das Stechmittel 6 in einem Reservoir 14, welches mit der auszubringenden Flüssigkeit gefüllt ist, vor und zurück bewegt. Das Füllen des Reservoirs 14 kann mittels Kapillarkräften erfolgen, indem die Aufnahme 5 zumindest teilweise in einen Flüssigkeitsvorrat getaucht wird. Eine rückseitige Öffnung 15 ist mittels einer das Stechmittel 6 umgreifenden Membrandichtung abgedichtet.

In diesem Fall erfolgt aus Ausbringen der Flüssigkeit aufgrund der adhäsionsgesteuerten Mitnahme der Flüssigkeit durch das Stechmittel 6. Allerdings ist diese Art des Ausbringens der Flüssigkeit weniger reproduzierbar, da die Flüssigkeit beim Eindringen des Stechmittels 6 an der Objektoberfläche teilweise abgestreift wird und somit unklar ist, welche Menge des Wirkstoffs tatsächlich an der "richtigen" Stelle platziert wurde. Bei einer weiteren Ausführung erfolgt die Flüssigkeitsabgabe zum Zeitpunkt des Erreichens einer maximalen Ausfahramplitude, wenn das Stechmittel 6 eine Solleinstechtiefe erreicht hat, indem auf das Reservoir 14 mit der auszubringenden Flüssigkeit ein Ausstoßimpuls gegeben wird, beispielsweise mittels einer Mikropumpe (nicht dargestellt), die an das Reservoir 14 gekoppelt ist. In einer alternativ Ausführung kann das Reservoir auch außerhalb der Aufnahme 5 gebildet sind, zum Beispiel als Außentank an dem Handgerät 1.

Die gewölbte Metalloberfläche der Aufnahme 5 bildet eine Messelektrode, während die Spitze 9 als Gegenelektrode wirkt. Die Einstechtiefe wird in der Messbetriebsart mittels einer Impedanzmessung gemessen. In Abhängigkeit von den Eigenschaften des Objektes im Einstechbereich können bestimmte Impedanzsprünge auftreten, die charakteristische Einstechtiefen anzeigen, die ihrerseits wiederum bestimmten Schichten des Objektes zuzuordnen sind.

Das Auftreten eines solchen charakteristischen Messwertes kann dann genutzt werden, um eine vorgegebene Ausfahramplitude für die Wirkstoffabgabe-Betriebsart festzulegen, wobei der Ausfahramplitude dem charakteristischen Messwert zugeordnete Ausfahramplitude zu diesem Zweck vergrößert oder verkleinert werden kann.

Die Stechbewegung des Stechmittels wird mit einer Einstechfrequenzen zwischen etwa 30Hz und etwa 250Hz sowie bevorzugt zwischen etwa 50Hz und etwa 200Hz ausgeführt. Bei gleichzeitiger translatorischer Bewegung des Handgerätes 1 über die Oberfläche des Objektes erfolgt das Einbringen der Flüssigkeit mit einer großen Zahl von Injektionen sehr schnell, so dass zum Beispiel eine Impfung nur einige Sekunden dauert. Wesentlich für einen sicheren Eintrag der Flüssigkeit ist dabei die präzise Einstellung der Einstechtiefe, die durch die Messung in der Messbetriebsart sowie eine geregelte Einstellung der Hubamplitude mittels des Steuereinrichtung 12 erfolgt.

Da häufig sehr geringe Einstechtiefen gewünscht sind, beispielsweise von etwa 50 µm bis wenige 100 µm, ist der Zeitpunkt der Flüssigkeitsabgabe sowie die sichere Handhabung des Handgerätes 1 für einen sicheren Angabeprozess kritisch. Werden für die energetische Umsetzung der Hubbewegung des Stechmittels 6 außer dem Stechmittel 6 selbst zusätzliche Massenkörper bewegt, führt dies zu Vibrationen oder Schwingungen längs und eventuell quer zur Hubbewegung, so dass kein sicherer Wirkstoffeintrag in der geforderten Tiefe möglich ist. Beispiele für solche gegebenenfalls weniger geeignete Antriebselemente in der Antriebseinrichtung 7 sind Schraubenantriebe, Kurbelantriebe, pneumatische und mechanische Antriebe zur Bewegung des Stechmittels 6. Diese Antriebe lassen sich wegen den zusätzlich zum Stechmittels 6 selbst bewegten großen Massen häufig weniger ausreichend vibrationsarm ausführen. Daher sind die Stechfrequenzen begrenzt. Da diese Antriebe zudem, aufgrund ihrer Bewegungsart, konstruktiv große Raumforderungen aufweisen, ist eine sichere Handhabe des Handgerätes 1 eventuell behindert.

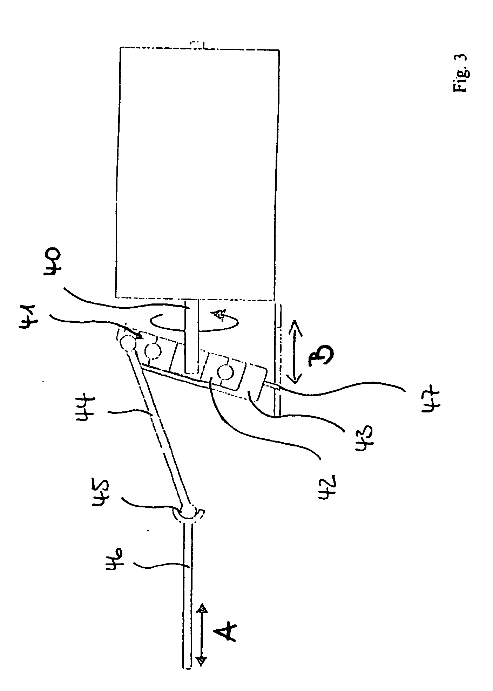

In einer bevorzugten Ausführung wird ein Taumelscheibenantrieb verwendet, mit dem eine Rotationsbewegung in eine repetierende Vor- und Zurückbewegung zum Antrieb des Stechmittels 6 umgesetzt wird. Fig. 3 zeigt einen Taumelscheibenantrieb im Querschnitt.

Fig. 3 eine schematische Darstellung eines Antriebsmechanismus, bei dem auf einer Antriebswelle 40 ein Kugellager 41 mit einem schräg auf der Antriebswelle 40 montierten Innenring 42 und einem freilaufenden Außenring 43 montiert ist. An dem Außenring 43 wird eine Pleuelstange 44 gehalten, die über ein Gelenk 45 mit einer Schubstange 46 gekoppelt ist. Beim Drehen der Antriebswelle 40 führt das Kugellager 41 eine taumelnde Bewegung aus, welche mit Hilfe der Pleuelstange 44 und der Schubstange 46 in eine lineare Antriebsbewegung für das Stechmittel in Pfeilrichtung A umgesetzt wird. Hierbei wird der Außenring 43 mit Hilfe einer Verdrehsicherung 47 festgehalten, die sich in Pfeilrichtung B vor und zurück bewegt.

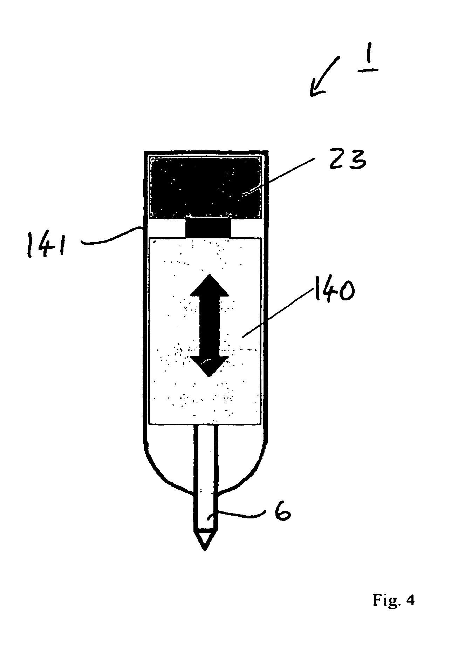

Durch die Bewegung des freilaufenden Außenringes 43 wird eine feste Ausfahramplitude des Stechmittels 6 erzeugt, zum Beispiel 2 mm. Die feste Ausfahramplitude lässt sich für den Stechvorgang mittels Verschiebung einer Antriebseinheit 140 mit dem Taumelscheibenantrieb im Gehäuse 141 des Handgerätes 1 relativ zur Öffnung 8 variabel gestalten, was in Fig. 4 schematisch gezeigt ist. Die Verschiebung der Antriebseinheit 140 im Gehäuse 141 bewirkt somit die Einstellung der gewünschten Einstechtiefe. Die Relativbewegung der Antriebseinheit 140 kann zum Beispiel durch den rotatorischen Antrieb 23 am oberen Ende des Handgerätes 1 erfolgen (Fig. 4). Die Sollwertvorgabe für die Einstechtiefe ergibt sich aus der Einstechtiefenmessung.

Zu Beginn des Prozesses zum kontrollierten Abgeben der Flüssigkeit wird die Ausfahramplitude des Stechmittels 6 zunächst sehr klein gewählt und dann in kleinen Schritten bis zu der erwünschten Einstechtiefe erhöht, die sich durch das Erreichen bestimmter Messwerte auszeichnet, beispielsweise einen Impedanzsprung. Bei allmählicher Zunahme der Amplitude bis zur Solleinstechtiefe erfordert einige Stechvorgänge. Bei jedem Einstechvorgang findet eine Impedanzmessung statt. Die Messwerte werden in einem der Messeinrichtung 10 zugeordneten Signalprozessor gespeichert. Ist das Stechmittel bis zu einer gewünschten Tiefe vorgedrungen, so tritt ein charakteristischer Messwert auf, beispielsweise ein erwarteter Impedanzwert. Die dann erreichte Ausfahramplitude kann für den weiteren Prozess festgehalten und in der Folge als Fixpunkt für die Einstechtiefe genutzt.

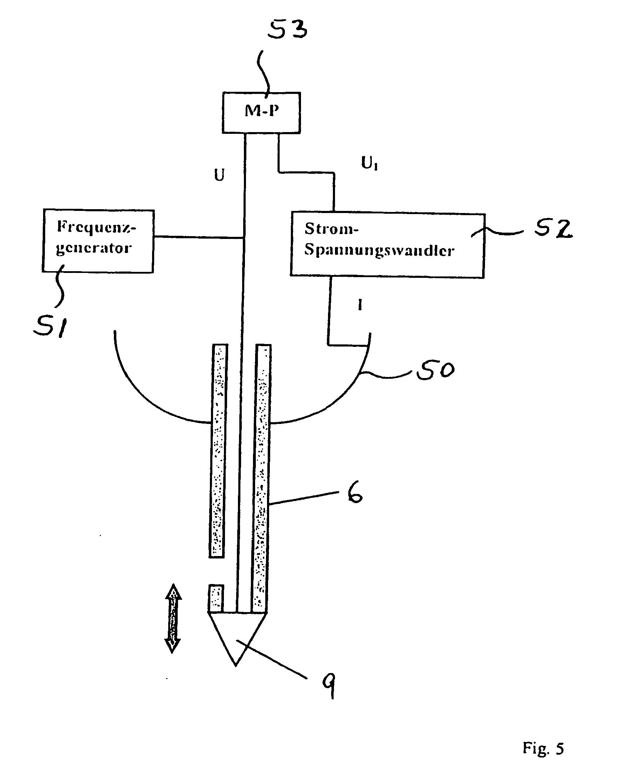

Fig. 5 zeigt eine schematische Darstellung eines Stechwerkzeugs mit hieran gekoppelter Messeinrichtung zum Messen einer Einstechtiefe von Stechmitteln.

Je nach Geräteausführung sind unterschiedliche Elektrodenvarianten zur Bestimmung der Einstechtiefe vorgesehen. So kann in einem Mehrnadelsystem beispielsweise vorgesehen sein, nur einen Teil der zum Aufstechen genutzten Nadeln auch als Elektrode in die Messung einbezogen wird. Es wird die Impedanz zwischen mindestens zwei Elektroden bestimmt, die bei der Ausführung in Fig. 5 mittels der Spitze 9 des Stechmittels 6, bei dem es sich um eine Hohlnadel handelt, und einem Abschnitt 50 der Aufnahme 5 gebildet sind. weiterhin ist ein Frequenzgenerator 51 vorgesehen. Zur Messung wird bevorzugt eine Wechselspannungsamplitude von höchstens 200mV zwischen den Elektroden eingesetzt. Der zwischen den Elektroden fließende Strom gelangt über einen Strom-Spannungswandler 52 als stromproportionales Spannungssignal in einen Signalprozessor 53, der zusammen mit dem Spannungssignal die Auswertung und Speicherung des Impedanzmesswerts vornimmt. Die Frequenz der Wechselspannung bewegt sich im Bereich von etwa 50kHz bis etwa 100kHz. Innerhalb dieser Frequenzspanne ist insbesondere eine sichere Diskriminierung zwischen Hautgewebe und Blut möglich, wenn der Wirkstoffeintrag in die Haut erfolgt.

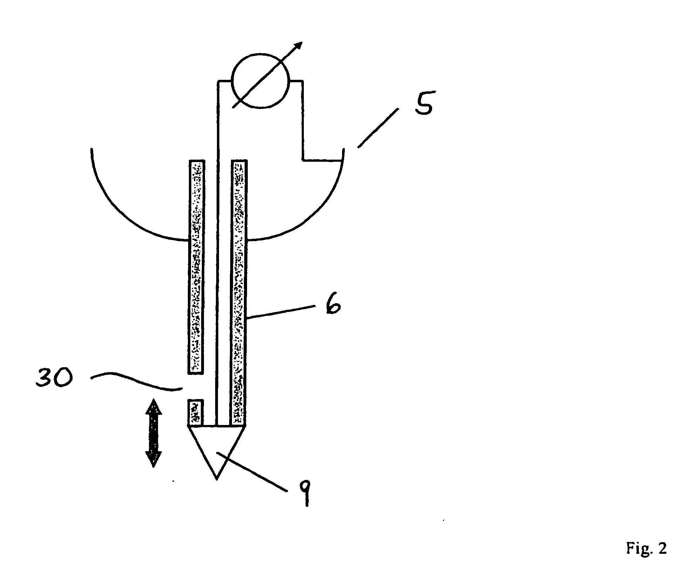

Bei der Ausführung in Fig. 2 wird das als Einzelnadel (Hohlnadel) ausgeführte Stechmittel 6 als eine Elektrode genutzt, insbesondere im Bereich der Spitze 9. Als Gegenelektrode und für den elektrischen Kontakt mit dem Objekt dient zur Impedanzmessung die metallische Aufnahme 5. Bei dem Einstechvorgang werden Strom und Spannung als Funktion der Einstechtiefe gemessen. Dringt das Stechmittel 6 in das Objekt ein, so ergibt sich aufgrund der Strom- und Spannungsmessung eine für das Objekt im Einstechbereich charakteristische Impedanz. Eine Auslassöffnung 30 für die Flüssigkeit ist in definiertem Abstand oberhalb der Spitze 9 gebildet. Die Auslassöffnung 30 ist in Fluidverbindung mit dem Reservoir (nicht dargestellt) für die auszubringende Flüssigkeit.

In der Messbetriebsart werden zur Ermittlung der Solleinstechtiefe bevorzugt nicht Absolutmesswerte sondern relative Änderung des Messwertes infolge des Auftretens charakteristischer Messwertsprünge untersucht. Bekanntermaßen ist zum Beispiel der Hautkontakt und damit auch die Präzision der Impedanzmessung sehr vom Oberflächenzustand der Haut abhängig, zum Beispiel durch die Absonderung von Schweiß. Untersuchungen haben gezeigt, dass beispielsweise eine Absolutmessung der Einstechtiefe in die Haut aufgrund ständig variierender Hautparameter häufig schwierig ist.

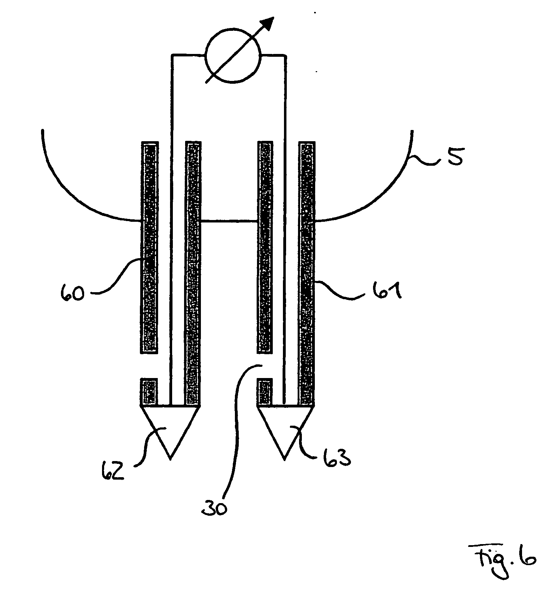

Gemäß Fig. 6 werden bei einem anderen Ausführungsbeispiel als Stechmittel zwei starr verbundene Stechnadeln 60, 61 genutzt, die gemeinsam in das Objekt eindringen, wobei Elektroden 62, 63 zur Impedanzmessung jeweils an der Spitze gebildet sind. Es können auch mehr als zwei Stechnadeln zum Einsatz kommen. Verglichen mit der vorherigen Ausführungsbeispiel liegt der Vorteil der Anordnung in Fig. 6 darin, dass mehrere Impfinjektionen gleichzeitig erfolgen, so dass der Impfprozess schneller beendet werden kann. Weiterhin lässt sich die Impedanzmessung zwischen mehreren Nadelelektroden ausführen, die parallel und gleichzeitig in das Objekt eindringen und nicht von dem Objektkontakt einer Elektrode abhängig sind, wodurch bessere und sichere Messwerte für die Einstechtiefe erzielbar sind.

In gleicher Weise wie bei der Messung mit einer Nadel wird zu Beginn des Impfprozesses die Eindringtiefe der Nadeln bis zum charakteristischen Sprung der Impedanz gesteigert. Die Ausfahramplitude der Stechbewegung wird sodann für den weiteren Impfprozess "eingefroren", bei dem wiederum der Wirkstoff durch multiple Injektionen verabreicht wird. Da in diesem Fall mehrere Nadeln für die Wirkstoffabgabe zur Verfügung stehen, verkürzt sich die Impfzeit gegenüber der Einnadellösung um den Faktor der Nadelanzahl.

Die Wirkstoffabgabe kann bei den verschiedenen Ausführungsformen in einfacher Weise durch das Eintauchen des Stechmittels in ein Reservoir für die Flüssigkeit bei der Rückbewegung erfolgen. Die Mitnahme der Flüssigkeit zur Abgabe erfolgt im einfachsten Fall durch Adhäsion der Flüssigkeit an der Nadel, insbesondere in Rillen, Vertiefungen oder Querbohrungen des Stechmittels, aber auch eine Nutzung von Nadeln mit einer glatten Oberfläche kann vorgesehen sein. In einer anderen Ausgestaltung sind die Stechmittel als Hohlnadel ausgeführt, wobei eine Auslassöffnung für die Flüssigkeit vorzugsweise an der Seite erfolgt, um eine Verstopfung des Hohlkörpers zu verhindern (vgl. Fig. 2 oben). Bei dieser Ausführung sind die Nadelbewegung sowie der Zeitpunkt der Flüssigkeitsabgabe fest gekoppelt.

Die Flüssigkeitsabgabe erfolgt in einer zweckmäßigen Ausführung beim Erreichen einer maximalen Eindringtiefe des Stechmittels, zum Beispiel durch einen Druckstoß einer "Mikropumpe" (nicht dargestellt). Zum Beispiel kann eine periodische Druckanregung der in einem elastisch deformierbaren Reservoirtank eingeschlossenen Flüssigkeit vorgesehen sein, beispielsweise mit einem kontrahierenden Piezoring, der synchron mit der Vorwärtsbewegung des Stechmittels Druck auf das Reservoir ausübt oder einem an die Stechmittelbewegung gekoppelten Stößel der die Druckanregung bewirkt.

Mit Hilfe der beschriebenen Vorrichtung ist ein gezieltes Einbringen von Wirkstoffen in ein Objekt ermöglicht, beispielsweise in die Haut. Langerhans'sche Zellen sind in der Stachelzellenschicht (*stratum spinosum*), am unteren Ende der Epidermis gelagert. Um sie optimal mit dem Impfstoff in Kontakt zu bringen, muss der Wirkstoff in der unmittelbaren Umgebung der Langerhans'schen Zellen freigesetzt werden und keineswegs in der unterhalb der Epidermis befindlichen Dermis. Bei einem Stechvorgang, bei dem die Injektionsnadel zu tief eindringt, besteht die Gefahr, dass der Wirkstoff in die papillare Dermis und damit in das Blut gelangt, oder in die Kollagenbündel der Dermis abgeleitet wird und somit seine Wirkung nicht entfalten kann. Genau dieses kann mit der vorangehend beschriebenen Vorrichtung vermieden werden.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Vorrichtung zum kontrollierten Einstechen in ein Objekt, insbesondere zum Aufstechen einer Haut, mit:
- einer Antriebseinrichtung, die konfiguriert ist, eine Antriebskraft zu liefern,
- einer Stecheinrichtung, die Stechmittel zum Einstechen in das Objekt umfasst und an die Antriebseinrichtung gekoppelt ist, sodass die Antriebskraft für eine repetierende Vor- / Rückwärtsbewegung der Stechmittel auf die Stechmittel eingeleitet ist,
- einer Messeinrichtung, die konfiguriert ist, in einer Messbetriebsart Messwerte für eine Einstechtiefe der Stechmittel in das Objekt zu ermitteln,
- einer Steuereinrichtung, die an die Messeinrichtung sowie die Antriebseinrichtung gekoppelt ist und konfiguriert ist, für eine Anwendungsbetriebsart eine Ausfahramplitude für die repetierende Vor- / Rückwärtsbewegung der Stechmittel einzustellen, welche einer vorgegebenen Einstechtiefe zugeordnet ist, die aus den in der Messbetriebsart ermittelten Messwerten für die Einstechtiefe abgeleitet ist,
**dadurch gekennzeichnet, dass** die Antriebseinrichtung die Antriebskraft für die repetierende Vor- / Rückwärtsbewegung der Stechmittel mit einer Frequenz zwischen etwa 30Hz und etwa 250Hz liefernd gebildet ist.

2. Vorrichtung nach Anspruch 1, **dadurch gekennzeichnet, dass** ein Reservoir mit einer einen Wirkstoff enthaltenden Flüssigkeit gebildet ist, welches mit den Stechmitteln durch eine Fluidverbindung verbunden ist, über die die Flüssigkeit zumindest in der Anwendungsbetriebsart zur kontrollierte Wirkstoffabgabe zu den Stechmitteln gelangt.

3. Vorrichtung nach Anspruch 1 oder 2, **dadurch gekennzeichnet, dass** die Messeinrichtung konfiguriert ist, um in der Messbetriebsart Messwerte für eine Einstechtiefe der Stechmittel in das Objekt gemäß zumindest einer der folgenden Messarten zu ermitteln: Einzeleinstichmessung und Messung von mehreren Einstichen bei repetierenden Vor- /Rückwärtsbewegungen der Stechmittel.

4. Vorrichtung nach Anspruch 2 oder 3, **dadurch gekennzeichnet, dass** die Fluidverbindung eine gepulst betreibbare Fluidverbindung ist, die konfiguriert ist, um in Abhängigkeit von einer Bewegungsfrequenz der repetierenden Vor- / Rückwärtsbewegung der Stechmittel die Flüssigkeit in Teilmengen aus dem Reservoir zu den Stechmitteln zu liefern.

5. Vorrichtung nach mindestens einem der Ansprüche 2 bis 4, **dadurch gekennzeichnet, dass** die Stechmittel durch einen Durchbruch in einer Wandung des Reservoirs geführt sind, in dem die Stechmittel bei der repetierenden Vor- / Rückwärtsbewegung geführt sind.

6. Vorrichtung nach mindestens einem der Ansprüche 2 bis 5, **gekennzeichnet durch** eine Pumpeinrichtung, die konfiguriert ist, um die Flüssigkeit aus dem Reservoir zu den Stechmitteln zu pumpen.

7. Vorrichtung Anspruch 6, **dadurch gekennzeichnet, dass** die Steuereinrichtung an die Pumpeinrichtung gekoppelt und konfiguriert ist, um einen die Pumpeinrichtung anregenden Pumpimpuls an die Pumpeinrichtung abzugeben, welcher einer vorgegebenen Ausfahramplitude einer ausgefahrenen Stellung der Stechmittel bei der repetierenden Vor- / Rückwärtsbewegung der Stechmittel zugeordnet ist.

8. Vorrichtung Anspruch 7, **dadurch gekennzeichnet, dass** die Steuereinrichtung konfiguriert ist, um den Pumpimpuls an die Pumpeinrichtung abzugeben, wenn die vorgegebene Amplitude einer maximal ausgefahrenen Stellung der Stechmittel entspricht.

9. Vorrichtung nach mindestens einem der Ansprüche 6 bis 8, **dadurch gekennzeichnet, dass** die Pumpeinrichtung eine Mikropumpe umfasst.

10. Vorrichtung nach mindestens einem der Ansprüche 2 bis 9, **dadurch gekennzeichnet, dass** die Fluidverbindung einen Kanal in den Stechmittel umfassen, welcher zu einer an den Stechmitteln gebildeten Abgabeöffnung führt.

11. Vorrichtung nach mindestens einem der vorangehenden Ansprüche, **dadurch gekennzeichnet, dass** die Messeinrichtung eine Impedanzmesseinrichtung zum Messen einer Impedanz des Objektes ist.

12. Vorrichtung nach mindestens einem der vorangehenden Ansprüche, **dadurch gekennzeichnet, dass** die Stechmittel eine oder mehrere an die Messeinrichtung gekoppelte Messelektroden umfassen.

13. Vorrichtung Anspruch 12, **dadurch gekennzeichnet, dass** mehrere Messelektroden jeweils durch eine von den Stechmitteln umfasste Nadel gebildet sind.

14. Vorrichtung Anspruch 12, **dadurch gekennzeichnet, dass** eine Messelektrode mittels der Stechmittel und eine Messgegenelektrode von einem Gehäuseteil gebildet sind.

15. Vorrichtung nach mindestens einem der vorangehenden Ansprüche, **dadurch gekennzeichnet, dass** die Stecheinrichtung in einem von der Antriebseinrichtung lösbaren Einwegmodul gebildet ist.

16. Vorrichtung nach mindestens einem der vorangehenden Ansprüche, **gekennzeichnet durch** einen elektrischen Impulsgenerator, welcher mit den Stechmitteln elektrisch verbunden ist und konfiguriert ist, um einen elektrischen Impuls auf die Stechmittel zu einem vorgegebenen Zeitpunkt zu geben.

17. Vorrichtung nach mindestens einem der vorangehenden Ansprüche, **dadurch gekennzeichnet, dass** die Antriebseinrichtung einen Wandlungsmechanismus umfasst, welcher konfiguriert ist, um eine Drehbewegung eines Elektromotors in eine auf die Stechmittel einzuleitende repetierende Schubbewegung zu wandeln.

18. Vorrichtung nach Anspruch 17, **dadurch gekennzeichnet, dass** der Wandlungsmechanismus einen Taumelscheibenanordnung umfasst, bei der eine Taumelscheibe an eine Antriebswelle des Elektromotors und an die Taumelscheibe ein Kopplungsmechanismus gekoppelt sind, wobei der Kopplungsmechanismus konfiguriert ist, um die repetierende Schubbewegung auf die Stechmittel einzuleiten.

19. Vorrichtung nach Anspruch 18, **dadurch gekennzeichnet, dass** der Kopplungsmechanismus einen an die Taumelscheibe gekoppelte Stößel aufweist, welcher in einer Linearführung geführt ist.

20. Vorrichtung nach mindestens einem der Ansprüche 2 bis 19, **dadurch gekennzeichnet, dass** das Reservoir in einer Stechmitteldüse gebildet ist.

21. Verfahren zum Betreiben einer Vorrichtung zum kontrollierten Einstechen in ein Objekt, bei dem von einer Stecheinrichtung umfasste Stechmittel zum Einstechen in das Objekt mittels einer Antriebseinrichtung, die eine Antriebskraft liefert, in eine repetierende Vor/ Rückwärtsbewegung versetzt werden, in einer Messbetriebsart mittels einer Messeinrichtung Messwerte für eine Einstechtiefe der Stechmittel in das Objekt gemessen werden und mittels einer Steuereinrichtung, die an die Messeinrichtung sowie die Antriebseinrichtung gekoppelt ist, für die repetierende Vor- / Rückwärtsbewegung der Stechmittel eine Ausfahramplitude für eine Anwendungsbetriebsart eingestellt wird, welche einer vorgegebenen Einstechtiefe zugeordnet ist, die aus den in der Messbetriebsart ermittelten Messwerten für die Einstechtiefe abgeleitet wird, **dadurch gekennzeichnet, dass** für die repetierende Vor- / Rückwärtsbewegung der Stechmittel eine Frequenz von etwa 30Hz bis etwa 250Hz eingestellt wird.

22. Verfahren nach Anspruch 21, **dadurch gekennzeichnet, dass** zumindest in einer Anwendungsbetriebsart zur kontrollierten Wirkstoffabgabe, welche sich an die Messbetriebsart anschließt, eine einen Wirkstoff enthaltende Flüssigkeit an die Stechmittel abgegeben wird.

23. Verfahren nach Anspruch 21, **dadurch gekennzeichnet, dass** die Flüssigkeit aus einem Reservoir über eine Fluidverbindung an die Stechmittel abgegeben wird.

24. Verfahren nach Anspruch 23, **dadurch gekennzeichnet, dass** die Flüssigkeit in Abhängigkeit von einer Bewegungsfrequenz der repetierenden Vor- / Rückwärtsbewegung der Stechmittel über eine gepulste Fluidverbindung in Teilmengen aus dem Reservoir zu den Stechmitteln geliefert wird.

25. Verfahren nach Anspruch 23 oder 24, **dadurch gekennzeichnet, dass** die Flüssigkeit unter Ausnutzung von Adhäsionskräften entlang eines Oberflächenabschnittes der Stechmittel geführt wird, indem die Stechmittel durch einen Durchbruch in einer Wandung des Reservoirs geführt sind, in welchem die Stechmittel bei der repetierenden Vor- / Rückwärtsbewegung geführt werden.

26. Verfahren nach mindestens einem der Ansprüche 23 bis 25, **dadurch gekennzeichnet, dass** die Flüssigkeit mittels einer Pumpeinrichtung aus dem Reservoir zu den Stechmitteln gepumpt wird.

27. Verfahren nach Anspruch 25, **dadurch gekennzeichnet, dass** die Steuereinrichtung an die Pumpeinrichtung gekoppelt und einen die Pumpeinrichtung anregenden Pumpimpuls an die Pumpeinrichtung abgibt, welcher einer vorgegebenen Ausfahramplitude einer ausgefahrenen Stellung der Stechmittel bei der repetierenden Vor- / Rückwärtsbewegung der Stechmittel zugeordnet ist.

28. Verfahren nach Anspruch 27, **dadurch gekennzeichnet, dass** die Steuereinrichtung den Pumpimpuls an die Pumpeinrichtung abgibt, wenn die vorgegebene Ausfahramplitude einer maximal ausgefahrenen Stellung der Stechmittel entspricht.

29. Verfahren nach mindestens einem der Ansprüche 21 bis 28, **dadurch gekennzeichnet, dass** mittels eines elektrischen Impulsgenerators, welcher mit den Stechmitteln elektrisch verbunden ist, ein elektrischer Impuls auf die Stechmittel zu einem vorgegebenen Zeitpunkt gegeben wird, wodurch an den Stechmitteln eine hohe elektrische Feldstärke erzeugt wird, wobei ein Impulsabgabezeitpunkt einer weiteren vorgegebenen Ausfahramplitude in einer weiteren ausgefahrenen Stellung der Stechmittel bei der repetierenden Vor- / Rückwärtsbewegung der Stechmittel zugeordnet wird, wobei die weitere vorgegebene Ausfahramplitude wahlweise mit der vorgegebnen Ausfahramplitude übereinstimmt.

30. Verfahren nach mindestens einem der Ansprüche 21 bis 29, **dadurch gekennzeichnet, dass** in der Antriebseinrichtung mit einem Wandlungsmechanismus eine Drehbewegung eines Elektromotors in eine auf die Stechmittel einzuleitende repetierende Schubbewegung umgewandelt wird.

## Claims

1. Device for the controlled piercing of an object, particularly the puncturing of a skin, with:
- a drive unit that is configured for the purpose of supplying a drive force,
- a piercing system comprising piercing means for piercing the object and which is coupled to the drive unit, so that the drive force for a repetitive forward / return movement of the piercing means is initiated and applied to the piercing means,
- a measuring system which is configured for the purpose of determining measuring values for a piercing depth of the piercing means into the object in a measuring operation mode,
- a control system coupled to the measuring system and to the drive unit and which is configured for the purpose of setting, for an application operation mode, an extension amplitude for the repetitive forward /return movement of the piercing means which are allocated to a pre-specified piercing depth that is derived from the measuring values determined in the measuring operation mode for the piercing depth, **characterized in that** the drive unit is configured to deliver the drive force for the repetitive forward / return movement with a frequency from about 30Hz to about 250Hz.

2. The device according to Claim 1, **characterized in that** a reservoir is formed with a liquid containing an active substance where said reservoir is connected to the piercing means by means of a fluid connection, by way of which the liquid makes its way to the piercing means at least in the application operation mode for the controlled discharge of active substance.

3. The device according to Claim 1 or 2, **characterized in that** the measuring system is configured for the purpose of determining, in the measuring operation mode, measuring values for a piercing depth of the piercing means in an object according to at least one of the following measuring modes: single-piercing measurement and measurement of several piercings for repetitive forwards/return movements of the piercing means.

4. The device according to Claim 2 or 3, **characterized in that** the fluid connection is a pulsed operable fluid connection which is configured for the purpose of supplying the liquid in partial volumes from the reservoir to the piercing means depending on a movement frequency of the repetitive forward / return movements of the piercing means.

5. The device according to at least one of the Claims 2 to 4, **characterized in that** the piercing means are routed through a breakthrough in a wall of the reservoir in which the piercing means for the repetitive forward / return movements of the piercing means are routed.

6. The device according to at least one of the Claims 2 to 5, **characterized by** a pump system that is configured for the purpose of pumping the liquid from the reservoir to the piercing means.

7. The device according to Claim 6, **characterized in that** the control system is coupled to the pump system and is configured for the purpose of transmitting a pump impulse, having an excitation effect on the pump system, to the pump system which is allocated to a pre-specified extension amplitude of a extended position of the piercing means during the repetitive forward / return movements of the piercing means.

8. The device according to Claim 7, **characterized in that** the control system is configured for the purpose of transmitting the pump impulse to the pump system if the pre-specified amplitude corresponds to a maximum extended position of the piercing means.

9. The device according to at least one of the Claims 6 to 8, **characterized in that** the pump system comprises a micro-pump.

10. The device according to at least one of the Claims 2 to 9, **characterized in that** the fluid connection comprises a channel in the piercing means, which leads to a discharge opening formed at the piercing means.

11. The device according to at least one of the preceding Claims, **characterized in that** the measuring system is an impedance measuring system for measuring an impedance of the object.

12. The device according to at least one of the preceding Claims, **characterized in that** the piercing means comprise one or several measuring electrodes coupled to the measuring system.

13. The device according to Claim 12, **characterized in that** several measuring electrodes are formed in each case by a needle enclosed by the piercing means.

14. The device according to Claim 12, **characterized in that** a measuring electrode is formed by means of the piercing means and a measuring counter electrode is formed by a casing part.

15. The device according to at least one of the preceding Claims, **characterized in that** the piercing system is formed in a disposable module detachable from the drive unit.

16. The device according to at least one of the preceding Claims, **characterized by** an electric impulse generator which is connected electrically to the piercing means and which is configured for the purpose of transmitting an electric impulse to the piercing means at a pre-specified point of time.

17. The device according to at least one of the preceding Claims, **characterized in that** the drive unit comprises a conversion mechanism which is for the purpose of converting a turning movement of the electric motor into a repetitive thrust movement to be applied to the piercing means.

18. The device according to Claim 17, **characterized in that** the conversion mechanism comprises a wobble disk arrangement in which a wobble disk is coupled to a drive shaft of the electric motor and a coupling mechanism is coupled to the wobble disk, where the coupling mechanism is configured for the purpose of initiating the repetitive thrust movement onto the piercing means.

19. The device according to Claim 18, **characterized in that** the coupling mechanism has a tappet that is coupled to the wobble disk, which tappet is positioned in a linear arrangement.

20. The device according to at least one of the Claims 2 to 19, **characterized in that** the reservoir is formed in a piercing means nozzle.

21. Method for operating a device for the controlled piercing of an object in which piercing means enclosed by a piercing system are put into a repetitive forward / return movement for piercing an object by means of a drive unit which supplies a force, in a measuring operation mode by means of a measuring unit, measuring values for a piercing depth of piercing means in an object are measured and, by means of a control system that is coupled to the measuring unit and the drive system, an extension amplitude for an application operation mode is set for the repetitive forward / return movement of the piercing means, which amplitude is allocated to a pre-specified piercing depth which is derived from measuring values for the piercing depth determined in the measuring operation mode, **characterized in that** a frequency from about 30Hz to about 250Hz is set of the repetitive forward / return movement of the piercing means.

22. The method according to Claim 21, **characterized in that** in at least one application operation mode for the controlled discharge of active substance, which follows the measuring operation mode, a liquid containing an active substance is discharged to the piercing means.

23. The method according to Claim 21, **characterized in that** the liquid from a reservoir is discharged to the piercing means by way of a fluid connection.

24. The method according to Claim 23, **characterized in that** the liquid is supplied by way of a pulsed fluid connection in partial volumes from the reservoir to the piercing means in depencence of a movement frequency of the repetitive forward / return movements of the piercing means.

25. The method according to Claim 23 or 24, **characterized in that** the liquid, with the usage of adhesion forces, is routed along a surface section of the piercing means where the piercing means are routed through a wall of the reservoir in which the piercing means for the forward / return movements of the piercing means are guided.

26. The method according to at least one of the Claims 23 to 25, **characterized in that** the liquid is pumped to the piercing means by means of a pump system.

27. The method according to Claim 25, **characterized in that** the control system is coupled to the pump system and transmits a pump impulse to the pump system, having an excitation effect on the pump system, which impulse is allocated to a pre-specified extension amplitude of an extended position of the piercing means during the repetitive forward / return movement of the piercing means.

28. The method according to Claim 27, **characterized in that** the control system transmits the pump impulse to the pump system if the pre-specified extension amplitude corresponds to a maximum extended position of the piercing means.

29. The method according to at least one of the Claims 21 to 28, **characterized in that,** by means of an electric impulse generator which is electrically connected to the piercing means, an electric impulse is transmitted to the piercing means at a pre-specified point of time, through which a high electric field strength is produced at the piercing means, where an impulse discharge time of a further pre-specified extension amplitude in a further extended position of the piercing means is allocated for the repetitive forward / return movements of the piercing means, where the further pre-specified extension amplitude selectively coincides with the pre-specified extension amplitude.

30. The method according to at least one of the Claims 21 to 29, **characterized in that,** in the drive unit, a turning movement of the electric motor is converted into a repetitive thrust movement to be initiated and applied to the piercing means with a converting mechanism.

## Revendications

1. Dispositif pour la piqûre contrôlée dans un objet, en particulier pour le perçage une peau, comprenant :
- un équipement d'entraînement qui est configuré pour fournir une force d'entraînement,
- un équipement de perforation qui comprend des moyens de perforation pour la piqûre dans l'objet et qui est couplé à l'équipement d'entraînement de manière à ce que la force d'entraînement soit appliquée aux moyens de perforation pour un mouvement en avant / en arrière répétitif des moyens de perforation,
- un équipement de mesure qui est configuré pour déterminer, dans un mode de mesure, des valeurs de mesure pour une profondeur de piqûre des moyens de perforation dans l'objet,
- un équipement de commande qui est couplé à l'équipement de mesure ainsi qu'à l'équipement d'entraînement et qui est configuré, pour un mode d'application, pour régler une amplitude de sortie pour le mouvement en avant / en arrière répétitif des moyens de perforation, laquelle amplitude est associée à une profondeur de piqûre prédéfinie qui est déduite des valeurs de mesure pour la profondeur de piqûre qui ont été déterminées dans le mode de mesure,
**caractérisé en ce que** l'équipement d'entraînement est formé de manière à fournir la force d'entraînement pour le mouvement en avant / en arrière répétitif des moyens de perforation à une fréquence comprise entre environ 30 Hz et environ 250 Hz.

2. Dispositif selon la revendication 1, **caractérisé en ce qu'**est formé un réservoir avec un liquide contenant un principe actif, lequel réservoir est relié aux moyens de perforation par une liaison fluidique via laquelle le liquide, au moins dans le mode d'application, parvient aux moyens de perforation en vue du débit contrôlé du principe actif.

3. Dispositif selon la revendication 1 ou 2, **caractérisé en ce que** l'équipement de mesure est configuré pour, dans le mode de mesure, déterminer des valeurs de mesure pour une profondeur de piqûre des moyens de perforation dans l'objet selon au moins l'un des modes de mesure suivants : mesure d'une piqûre unique et mesure de plusieurs piqûres lors de mouvements en avant / en arrière répétitifs des moyens de perforation.

4. Dispositif selon la revendication 2 ou 3, **caractérisé en ce que** la liaison fluidique est une liaison fluidique pouvant être exploitée de manière pulsée qui est configurée pour, en fonction d'une fréquence de mouvement du mouvement en avant / en arrière répétitif des moyens de perforation, fournir le liquide aux moyens de perforation en quantités partielles depuis le réservoir.

5. Dispositif selon au moins l'une des revendications 2 à 4, **caractérisé en ce que** les moyens de perforation sont guidés à travers une percée dans une paroi du réservoir, dans laquelle les moyens de perforation sont guidés lors du mouvement en avant / en arrière répétitif.

6. Dispositif selon au moins l'une des revendications 2 à 5, **caractérisé par** un équipement de pompage qui est configuré pour pomper le liquide du réservoir vers les moyens de perforation.

7. Dispositif selon la revendication 6, **caractérisé en ce que** l'équipement de commande est couplé à l'équipement de pompage et configuré pour délivrer à l'équipement de pompage une impulsion de pompage qui excite l'équipement de pompage, laquelle impulsion est associée à une amplitude de sortie prédéfinie d'une position sortie des moyens de perforation lors du mouvement en avant / en arrière répétitif des moyens de perforation.

8. Dispositif selon la revendication 7, **caractérisé en ce que** l'équipement de commande est configuré pour délivrer l'impulsion de pompage à l'équipement de pompage quand l'amplitude prédéfinie correspond à une position sortie au maximum des moyens de perforation.

9. Dispositif selon au moins l'une des revendications 6 à 8, **caractérisé en ce que** l'équipement de pompage comprend une micropompe.

10. Dispositif selon au moins l'une des revendications 2 à 9, **caractérisé en ce que** la liaison fluidique comprend dans les moyens de perforation un canal qui mène à une ouverture de débit formée sur les moyens de perforation.

11. Dispositif selon au moins l'une des revendications précédentes, **caractérisé en ce que** l'équipement de mesure est un équipement de mesure d'impédance pour mesurer une impédance de l'objet.

12. Dispositif selon au moins l'une des revendications précédentes, **caractérisé en ce que** les moyens de perforation comprennent une ou plusieurs électrodes de mesure couplées à l'équipement de mesure.

13. Dispositif selon la revendication 12, **caractérisé en ce que** plusieurs électrodes de mesure sont formées chacune par une aiguille entourée par les moyens de perforation.

14. Dispositif selon la revendication 12, **caractérisé en ce qu'**une électrode de mesure est formée par les moyens de perforation et une contre-électrode de mesure par une partie de boîtier.

15. Dispositif selon au moins l'une des revendications précédentes, **caractérisé en ce que** l'équipement de perforation est formé dans un module à usage unique détachable de l'équipement d'entraînement.

16. Dispositif selon au moins l'une des revendications précédentes, **caractérisé par** un générateur d'impulsions électriques qui est relié électriquement aux moyens de perforation et qui est configuré pour délivrer une impulsion électrique aux moyens de perforation à un instant prédéfini.

17. Dispositif selon au moins l'une des revendications précédentes, **caractérisé en ce que** l'équipement d'entraînement comprend un mécanisme de conversion qui est configuré pour convertir un mouvement de rotation d'un moteur électrique en un mouvement de translation répétitif à appliquer aux moyens de perforation.

18. Dispositif selon la revendication 17, **caractérisé en ce que** le mécanisme de conversion comprend un arrangement à plateau oscillant dans lequel un plateau oscillant est couplé à un arbre d'entraînement du moteur électrique et un mécanisme de couplage est couplé au plateau oscillant, le mécanisme de couplage étant configuré pour appliquer le mouvement de translation répétitif aux moyens de perforation.

19. Dispositif selon la revendication 18, **caractérisé en ce que** le mécanisme de couplage présente un poussoir couplé au plateau oscillant, lequel poussoir est guidé dans un guide linéaire.

20. Dispositif selon au moins l'une des revendications 2 à 19, **caractérisé en ce que** le réservoir est formé dans une buse de moyen de perforation.

21. Procédé pour exploiter un dispositif pour la piqûre contrôlée dans un objet, dans lequel on imprime un mouvement en avant / en arrière répétitif à des moyens de perforation compris dans un équipement de perforation au moyen d'un équipement d'entraînement qui fournit une force d'entraînement, on mesure, dans un mode de mesure, au moyen d'un équipement de mesure des valeurs de mesure pour une profondeur de piqûre des moyens de perforation dans l'objet et on règle, pour un mode d'application, au moyen d'un équipement de commande qui est couplé à l'équipement de mesure ainsi qu'à l'équipement d'entraînement, une amplitude de sortie pour le mouvement en avant / en arrière répétitif des moyens de perforation, laquelle amplitude est associée à une profondeur de piqûre prédéfinie qui est déduite des valeurs de mesure pour la profondeur de piqûre qui ont été déterminées dans le mode de mesure, **caractérisé en ce que** l'on règle, pour le mouvement en avant / en arrière répétitif des moyens de perforation, une fréquence comprise entre environ 30 Hz et environ 250 Hz.

22. Procédé selon la revendication 21, **caractérisé en ce que,** au moins dans un mode d'application servant au débit contrôlé d'un principe actif qui fait suite au mode de mesure, un liquide contenant un principe actif est délivré aux moyens de perforation.

23. Procédé selon la revendication 21, **caractérisé en ce que** le liquide est délivré aux moyens de perforation depuis un réservoir via une liaison fluidique.

24. Procédé selon la revendication 23, **caractérisé en ce que** le liquide est fourni aux moyens de perforation en quantités partielles depuis le réservoir, via une liaison fluidique pulsée, en fonction d'une fréquence de mouvement du mouvement en avant / en arrière répétitif des moyens de perforation.

25. Procédé selon la revendication 23 ou 24, **caractérisé en ce que** le liquide est guidé le long d'une portion de surface des moyens de perforation en utilisant des forces d'adhérence, les moyens de perforation étant guidés à travers une percée dans une paroi du réservoir dans laquelle les moyens de perforation sont guidés lors du mouvement en avant / en arrière répétitif.

26. Procédé selon au moins l'une des revendications 23 à 25, **caractérisé en ce que** le liquide est pompé du réservoir vers les moyens de perforation au moyen d'un équipement de pompage.

27. Procédé selon la revendication 25, **caractérisé en ce que** l'équipement de commande est couplé à l'équipement de pompage et délivre à l'équipement de pompage une impulsion de pompage qui excite l'équipement de pompage, laquelle impulsion est associée à une amplitude de sortie prédéfinie d'une position sortie des moyens de perforation lors du mouvement en avant / en arrière répétitif des moyens de perforation.

28. Procédé selon la revendication 27, **caractérisé en ce que** l'équipement de commande délivre l'impulsion de pompage à l'équipement de pompage quand l'amplitude de sortie prédéfinie correspond à une position sortie au maximum des moyens de perforation.

29. Procédé selon au moins l'une des revendications 21 à 28, **caractérisé en ce que,** au moyen d'un générateur d'impulsions électriques qui est relié électriquement aux moyens de perforation, une impulsion électrique est délivrée aux moyens de perforation à un instant prédéfini, suite à quoi une intensité de champ électrique élevée est générée aux moyens de perforation, un instant de délivrance d'impulsion étant associé à une autre amplitude de sortie prédéfinie dans une autre position sortie des moyens de perforation lors du mouvement en avant / en arrière répétitif des moyens de perforation, l'autre amplitude de sortie prédéfinie concordant au choix avec l'amplitude de sortie prédéfinie.

30. Procédé selon au moins l'une des revendications 21 à 29, **caractérisé en ce que,** dans l'équipement d'entraînement, un mouvement de rotation d'un moteur électrique est converti par un mécanisme de conversion en un mouvement de translation répétitif à appliquer aux moyens de perforation.
